# EUROPEAN PATENT APPLICATION

(11) **EP 1 906 296 A2**
(43) Date of publication of application: **02.04.2008**
(21) Application number: 07018354.6
(22) Date of filing: 19.09.2007
(51) Int. Cl.: G06F 3/01

(54) **Method of employing a gaze direction tracking system for control of a computer**

(30) Priority: 27.09.2006 GB 0618978
(71) Applicant: Malvern Scientific Solutions Limited, Suckley Worcestershire WR6 5DR (GB)
(72) Inventor: Beale, Marc, Ivor, John, Suckley Worcestershire WR6 5DR (GB)
(74) Representative: Jackson, Derek Charles

(57) **Abstract**

A method of employing a gaze direction tracking system for control of a computer comprises the steps of: providing a computer display incorporating a screen and at least one off-screen control target (1, 2, 3, 4); and eye-pointing by a user at the control target. The eye-pointing is detected by the gaze control tracking system so as to effect a predetermined control action in the computer.

## Description

This invention relates to a method of employing gaze direction tracking system for control of a computer, such as a personal computer or assistive technology for a user with a disability.

Gaze direction tracking offers the potential for a user to control software on a personal computer simply by looking at the computer's display. However, there are problems which frustrate users of known gaze direction tracking systems. Firstly, there is the need for the user's eyes both to view the display (acting as an "input device") and to make selections (acting as an "output device"), and secondly there is inaccuracy in the measured location of eye-pointing.

The need for the user's eyes both to view the display and to make selections is associated with, for example, the pop-up on-screen menus of existing gaze direction trackers which many users find difficult to operate, particularly if unfamiliar with gaze direction tracking or if the user has a cognitive disability. It is also desirable to avoid cluttering a computer display with elements which could interfere with the normal use of computer software.

Inaccuracy is an inherent problem in gaze direction tracking. The position sensing accuracy is generally not good, with an offset error (i.e., the difference between an actual cursor position on the display compared with an intended cursor position) of typically up to 10 mm or more, which is insufficiently accurate to operate most mainstream applications software. Many mainstream applications software programs require an accuracy of a few millimetres, while some require pixel-placement accuracy.

Even an accuracy of about 10 mm assumes that the user has undergone a calibration procedure. This may be inconvenient or even impossible in some circumstances, for example if members of the public use gaze direction tracking to access banking facilities, or when a disabled user does not have the cognitive ability to undergo the calibration procedure. Without the calibration procedure, the positional accuracy of gaze direction tracking is generally significantly worse than about 10 mm.

If a gaze direction tracking system is used to control the position of a 'live' cursor (the position of which is generally updated many times per second according to the direction of eye pointing) the resulting offset error makes it very difficult to control the cursor position accurately. Any attempt by the user to correct for offset error by re-directing his or her eyes to a position where the cursor should be placed makes the use of live cursor positioning very inconvenient.

Magnification of part of the computer display can be used to improve the relative accuracy of gaze direction tracking systems and reduces the severity of the problem. However, this approach is in itself insufficient because the offset error remains significant at practicable magnifications and continues to frustrate the user. With simple magnification alone, it remains difficult to use mainstream applications software with gaze direction tracking systems.

As a result, the use of gaze direction tracking systems is generally restricted to software designed for disabled users in which low pointing precision is required because the user only selects from an array of relatively large cells and the effect of offset error is reduced.

Improvement to the fundamental accuracy of gaze location would be of considerable benefit to the user, particularly if the need to undergo a calibration procedure could be eliminated.

It is therefore an object of the present invention to provide a method of employing a gaze direction tracking system for control of a computer which overcomes or at least ameliorates at least one of the above disadvantages.

According to the present invention there is provided a method of employing a gaze direction tracking system for control of a computer comprising the steps of: providing a computer display incorporating a screen and at least one off-screen control target; and eye-pointing by a user at the control target, whereby the eye-pointing is detected by the gaze control tracking system so as to effect a predetermined control action in the computer.

The control target may include means for indicating that a user has eye-pointed at the target and that the predetermined control action has been effected. The indicating means may comprise at least one light emitting diode. The indicating means may be illuminated to indicate that the predetermined control action has been effected and/or may change colour.

The at least one control target may include a target for engaging and disengaging the gaze control tracking system.

The at least one control target may include manually-operable switch means.

The method may include the step of calibrating the gaze control tracking system by eye-pointing to a point on the screen so as to cause a first marker to appear on the screen at a calculated location of eye-pointing and to cause a second marker to appear on the screen at a location offset from the first marker, and subsequently eye-pointing at the second marker so as to enable the gaze control system to calculate and update the location of the first marker. When re-located, the first marker may take the same or a different form..

At least one further control target may be provided on the screen, for example in graphical form, such as for controlling the function of software running on the computer.

The method may include the step of dynamically re-calibrating the gaze control system by applying a positional correction based on previously acquired calibration data. The calibration data may be acquired whilst the user was gazing at a predetermined location within a predetermined time prior to the positional correction being applied. The predetermined location may be a control target. The predetermined location may be within substantially 100 mm, preferably within substantially 50 mm, of the previous gaze position of the user. The predetermined time may be within substantially one minute and may preferably be within substantially ten seconds and more preferably within substantially two seconds.

For a better understanding of the present invention and to show more clearly how it may be carried into effect reference will now be made, by way of example, to the accompanying drawings in which:
Figure 1 shows a computer display for use with the method according to the present invention;
Figure 2 shows a screen of a computer display according to one embodiment of the present invention after a user has made an initial target selection; and
Figure 3 shows an on-screen display with controls which can be used for dynamic correction of gaze direction tracking.

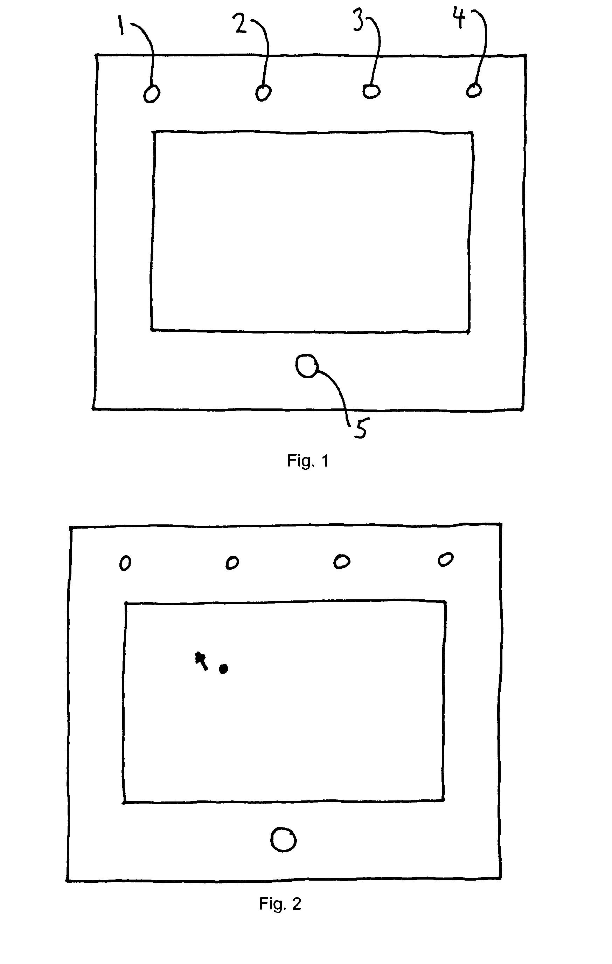

The computer display 1 shown in Figure 1 incorporates four light emitting diodes (LEDs) arranged at the top of a screen together with a gaze direction tracking camera 5 located at the bottom of the screen. The state of illumination and/or colour of the LEDs provides feedback on the status of a gaze direction control system. The off-screen controls provide the user with control over the gaze direction tracker. For example, an LED 2 at the top left-centre of the display is normally red to signify the system is disengaged and the user can then view the display on the screen without any further actions occurring. On gazing at the LED 2, it turns green to indicate the system is engaged and will respond to the user eye-pointing to a target on the screen. When the user gazes once again at the LED 2 while it is green, the system is disengaged and the LED reverts to red. Alternatively or additionally, the green LED may revert to red and the system may be disengaged once an action has been selected by eye-pointing as will be described hereinafter or after a predetermined time-out period.

The LED is therefore used as a target for engaging the gaze direction tracking system and is located a sufficient distance (for example, about 30 to 50 mm) outside the edge of the computer's screen to avoid inadvertent triggering when viewing items on the screen. The LED target is also determined by the system to have a relatively large target area, such as a square of 25 to 30 mm by 25 to 30 mm, to make engagement of the system a simple operation not requiring precise eye-pointing. Of course the size and shape of the target area may be different if desired. The system is then engaged by gazing at the target area for a predetermined time, for example about a quarter to half a second, whereupon the system is engaged and remains active until either it is positively disengaged by the user or there is no relevant activity for a predetermined time.

Additional LEDs may be provided to offer further functionality, for example when used with disabilities-focussed software which provides the user with access to text, photographs, music and typing, using multiple grids of options, that is menus. For example, when reading an on-screen text, eye-pointing at LED 4 may be used to "turn the page" and looking at LED1 may be used to "turn back a page". LEDs 1 and 4, which are positioned at opposite sides of the top of the display, may have alternative functions with different aspects of the software. For example, when listening to music LED 4 may move to the next track, while LED 1 may return to the previous track. As a further alternative, LEDs 1 and 4 may be used to the previous or to the next menu of options. The LEDs themselves may be switched to on to indicate when they are active and to off to indicate when they are inactive.

LED 3, at the top right-centre of the display, may enable the user to return to a specific point in the software, such as the "home page" of the disabilities-focussed software.

The LEDs may be colour coded to assist the user in identifying their different functions. For example, LEDs 1 and 4 may be illuminated amber to indicate moving to another display, while LED 3 may be illuminated blue to indicate resetting the software to the "home page".

Switches (not shown) may be associated with each LED to enable the user, or an assistant, to operate the controls manually.

Once the system has been engaged, the user eye-points to a desired location on the screen. The result of this is shown in one embodiment in Figure 2. The conventional system cursor is shown at the (known) location where the gaze tracking system calculates the point of gaze to be, with inherent inaccuracies, and also a 'dot' which is offset from, but near to, the cursor. The user then redirects his gaze at the 'dot' which allows the gaze tracking system to acquire the data it needs to calibrate itself. The system cursor and the 'dot' are removed from the screen and are replaced at the location of eye-pointing as determined from the new data either in the form of the system cursor or as an alternative cursor, such as a cross-hair cursor.

The absolute accuracy of eye-pointing can be enhanced as described above by applying a positional correction based on the measured data obtained when the user is directing his or her gaze at a known location. The use of a one-off calibration procedure of this type is well-known with gaze direction tracking. The first time a user uses the gaze direction tracker, the system is calibrated for that user, with the user looking at one or more on-screen targets of known location.

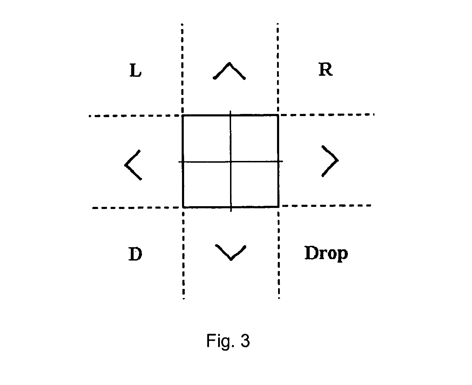

According to an aspect of the present invention, an alternative or additional dynamic calibration procedure takes place while the user is using the system in order to further enhance the accuracy of eye-pointing. That is, the present invention takes advantage of the fact that the user frequently directs his or her eyes at locations of known position (a known target), such as the 'dot' in Figure 2 or the controls of Figure 3 which are additional to those of Figures 1 and 2. In Figure 3, a graphical display is provided on the screen with a central cross-hair cursor surrounded by eight squares containing controls, such as arrows to indicate a desired direction of cursor movement and indicia, such as L, R, D and Drop for indication mouse control actions such as left click, right click, double click, and drag and drop The controls are provided at predetermined locations on the screen and are therefore known targets. Selection of any of these controls provides events which in turn provide calibration data which can be used to enhance the accuracy of eye-pointing to a predetermined target of unknown location (an unknown target). It is also possible to use the off-screen controls, but accuracy may not be as good because of the greater separation from on-screen targets.

Such a dynamic calibration procedure significantly enhances the accuracy of eye-pointing because it takes place locally both in time and space. That is, it typically occurs only a few seconds before a selection is made, during which time the user's eye position and condition of tear fluid will have changed very little.

Ideally, dynamic calibration should be effected as soon as possible before a selection is made, most preferably within less than one second, and as close as possible to the location of the selected target, most preferably less than 40 mm.

In practice, the time between eye-pointing to an unknown target and then eye-pointing to a known target will be a few seconds, typically less than two seconds, and provides a significant dynamic enhancement in accuracy compared with a conventional calibration procedure which will have been undertaken a considerable time, possibly even days, before.

In practice, dynamic calibration also occurs relatively close to the location of the unknown target on the screen. In this way, the user makes only a small movement of head or eyes when moving from the known target to the unknown target. The spatial separation between the known target and the unknown target is ideally less than 100 mm and preferably less than about 50 mm. The or each known target may be provided on the computer screen, such as one or more of the on-screen controls. Alternatively, the or each known target may be located remotely from the screen, such as offset below the lower edge of the screen or offset above the upper edge of the screen, although the spatial separation between the known target and the unknown target will be somewhat greater.

Error correction may be employed, for example, as a number of pixels in the x and y directions. Then if the user is required to gaze at a target having actual pixel co-ordinates of x and y, then the gaze direction tracking system will return a measured position of x±Δx and y±Δy. Subsequently the user eye-points to a desired unknown target having co-ordinates of X and Y, the gaze direction tracker will return a measured position of X±ΔX and Y±ΔY. Since it is assumed that the two errors Δx and ΔX and the two errors Δy and ΔY are the same, the measured location of the unknown target can be correct, as will be familiar to one skilled in the art. It has been found that such a procedure significantly improves the accuracy of gaze direction tracking.

The gaze direction tracking system described above may be modified by positioning all the controls other than on the computer screen and spaced from the edges of the screen.

## Claims

1. A method of employing a gaze direction tracking system for control of a computer comprising the steps of: providing a computer display incorporating a screen and at least one off-screen control target (1, 2, 3, 4); and eye-pointing by a user at the control target, whereby the eye-pointing is detected by the gaze control tracking system so as to effect a predetermined control action in the computer.

2. A method according to claim 1, **characterised in that** the control target (1, 2, 3, 4) includes means for indicating that a user has eye-pointed at the target and that the predetermined control action has been effected.

3. A method according to claim 2, **characterised in that** the indicating means comprises at least one light emitting diode.

4. A method according to claim 2 or 3, **characterised in that** the indicating means is illuminated to indicate that the predetermined control action has been effected.

5. A method according to claim 2, 3 or 4, **characterised in that** the indicating means changes colour to indicate that the predetermined control action has been effected.

6. A method according to any preceding claim, **characterised in that** the at least one control target (1, 2, 3, 4) includes a target for engaging and disengaging the gaze control tracking system.

7. A method according to any preceding claim, **characterised in that** the at least one control target (1, 2, 3, 4) includes manually-operable switch means.

8. A method according to any preceding claim, **characterised in that** it includes the step of calibrating the gaze control tracking system by eye-pointing to a point on the screen so as to cause a first marker to appear on the screen at a calculated location of eye-pointing and to cause a second marker to appear on the screen at a location offset from the first marker, and subsequently eye-pointing at the second marker so as to enable the gaze control system to calculate and update the location of the first marker.

9. A method according to any preceding claim, **characterised in that** at least one further control target is provided on the screen, such as for controlling the function of software running on the computer.

10. A method according to claim 9, **characterised in that** the at least one further control target is provided in graphical form.

11. A method according to any preceding claim, **characterised in that** it includes the step of re-calibrating the gaze control system by applying a positional correction based on previously acquired calibration data.

12. A method according to claim 11, **characterised in that** the calibration data is acquired whilst the user was gazing at a predetermined location within a predetermined time prior to the positional correction being applied.

13. A method according to claim 12, **characterised in that** the predetermined location is a control target.

14. A method according to claim 12 or 13, **characterised in that** the predetermined location is within substantially 100 mm of the previous gaze position of the user.

15. A method according to claim 14, **characterised in that** the predetermined location is within substantially 50 mm of the previous gaze position of the user.

16. A method according to any one of claims 12 to 15, **characterised in that** the predetermined time is within substantially one minute.

17. A method according to claim 16, **characterised in that** the predetermined time is within substantially ten seconds.

18. A method according to claim 17, **characterised in that** the predetermined time is within substantially two seconds.
